# EUROPEAN PATENT SPECIFICATION

(11) **EP 2 981 297 B1**
(45) Date of publication and mention of the grant of the patent: **03.05.2017**
(21) Application number: 14722352.3
(22) Date of filing: 31.03.2014
(51) Int. Cl.: A61L 2/06, B65B 55/06

(54) **STERILISING SYSTEM**
STERILISIERUNGSSYSTEM
SYSTÈME DE STÉRILISATION

(30) Priority: 05.04.2013 IT PR20130031
(43) Date of publication of application: 10.02.2016
(73) Proprietor: G.F. S.p.A., 43040 Solignano (Parma) (IT)
(72) Inventor: SERVENTI, Franco, I-43040 Varano Dè Melegari (IT)
(74) Representative: Monelli, Alberto
(86) International application number: PCT/IB2014/060319
(87) International publication number: WO 2014/162251

(56) References cited:
- WO-A1-02/051450
- US-A1- 2005 180 900
- US-A1- 2008 181 826

## Description

### Technical field

The object of the present invention is a sterilising system. This system is utilised for the sterilisation of containers, typically glass containers, preferably phials or vials.

### Background art

There are known sterilising systems that comprise a tunnel provided with a chamber in which an air flow is heated by means of a set of electric heating elements and the air flow then heats the phials or vials positioned in said chamber. The heating process is carried out according to a precise temperature profile so as to ensure sterilisation.

However, one drawback of this type of design relates to the management costs of the system, considering the high level of electric energy consumption determined by the electric heating elements.

An additional drawback is related to the fact that the heating elements are powered with high voltage and therefore the safety of the system must be managed with extreme care, especially during maintenance procedures. There are also known sterilising systems illustrated in the patent documents WO02/051450, US2008/181826 and US2005/180900.

### Disclosure of the invention

In this context, the technical task underlying the present invention is to offer a sterilising system that allows for a reduction in operating costs. A further important aim of the present invention is to make available a sterilising system that makes it possible to improve the safety of operators. The defined technical task and the specified aims are substantially achieved by a sterilising system comprising the technical characteristics set forth in one or more of the appended claims.

### Brief description of drawings

Further characteristics and advantages of the present invention will become more apparent from the indicative and thus non-limiting description of a preferred, but not exclusive embodiment of an exchanger, as illustrated in the accompanying drawings, in which:

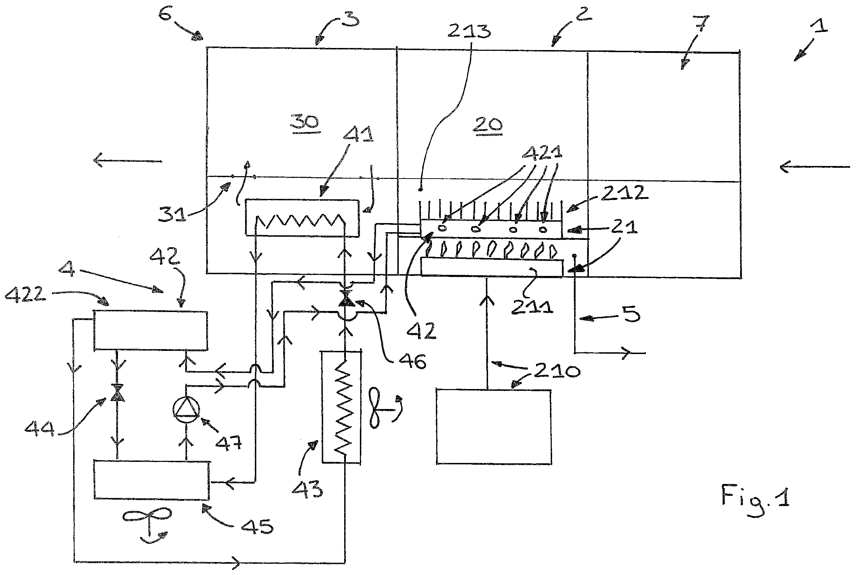

-Figure 1 shows a schematic view of the system.

### Detailed description of preferred embodiments of the invention

A system for sterilising containers, typically glass containers and preferably designed for containing pharmaceutical products, is indicated in the accompanying figures by the reference number 1. Appropriately, these containers consist of phials and/or vials.

This system 1 is suitably located downstream of a washing machine for washing the containers. The system 1 is suitably located upstream of a filling machine. The washing machine performs a preliminary washing procedure, whereas the filling machine is responsible for introducing the product in the containers.

The system 1 suitably comprises a storage chamber 7 for storing the containers. The chamber 7 comprises introducing means for introducing a fluid flow (typically sterile air) into said chamber 7. Advantageously, this fluid flow makes it possible to dry moisture present on the containers after the washing procedure carried out in the washing machine. Advantageously, this fluid flow is disposed of.

Moreover, the chamber 7 comprises packing means for packing the containers compactly. It is important that the containers be packed compactly and evenly distributed in the chamber 7; in fact, this manner they can be advanced along the system 1 in an organised fashion (and this has positive effects on the uniform distribution of the heat).

The system 1 comprises a sterilising chamber 2. This chamber 2 is advantageously a depyrogenation chamber (depyrogenation being a strong form of sterilisation). The sterilising chamber 2 is located downstream of the chamber 7 (with respect to the direction of advancement of the containers). The sterilising chamber 2, in turn, comprises a positioning zone 20 for positioning of the containers.

The system 1 comprises heating means 21 for heating a first fluid flow that envelops the containers placed in said positioning zone 20 so as to sterilise them (or preferably to carry out depyrogenation thereof).

The heating means 21 preferably but not necessarily are external to the positioning zone 20. The heating means 21 heat the first fluid flow (which transits in the positioning zone 20).

The system 1 suitably comprises a cooling chamber 3 for cooling the containers. The cooling chamber 3 is located downstream of the sterilising chamber 2 with respect to the direction of advancement of the containers. The system 1 comprises supply means 210 for supplying oxyhydrogen (also known as "Brown's gas"). The supply means 210 usually comprises an oxyhydrogen generator (but it could comprise an oxyhydrogen accumulator). The use of oxyhydrogen as fuel is extremely advantageous, as it makes it possible to obtain a flame that is extremely hot and in addition, the combustion thereof generates water vapour (but not toxic residues).

The means 21 for heating the containers present in the sterilising chamber 2 comprises an oxyhydrogen burner 211 located downstream of the supply means 210 (with respect to the flow of oxyhydrogen).

The heating means 21 also comprises a heat sink 212; said heat sink 212 is heated by said burner 211 and is in thermal communication with said positioning zone 20 for the positioning of the containers in the sterilising chamber 2. Said heat sink 212 (or at least part of the heating means 21) is found in a first compartment 213 external to the positioning zone 20 for the positioning of the containers in the sterilising chamber 2. The system 1 comprises a filter that separates said first compartment 213 from said positioning zone 20 for the positioning of the containers. The first fluid flow travelling through said filter so as to pass from the first compartment 213 to the positioning zone 20. Suitably, the first fluid flow flowing out of the first compartment 213 is introduced into the positioning zone 20 and then recirculated in said first compartment 213.

The heat sink 212 is suitably made of aluminium or aluminium alloy. The Applicant has experimentally found that in addition to having an optimal coefficient of heat exchange, this material offers high resistance to the oxyhydrogen flame (this minimises the risk of the oxyhydrogen flame ruining the heat sink 212). Suitably, the heat sink 212 is finned so as to improve the heat exchange with the first fluid flow.

The heat sink 212 could also be made of another material, such as stainless steel for example.

The system 1 comprises a refrigeration circuit 4 that subtracts heat from the cooling chamber 3. This circuit 4 is important for the purpose of bringing about cooling of the containers previously heated in the sterilising chamber 2. In fact, before filling the containers, prior cooling of the containers at 40-50 °C is convenient (in order to prevent the containers from damaging the product that is inserted therein). Suitably, the circuit 4 is an absorption refrigeration circuit 4. Generally speaking, absorption circuits are known in the art. The circuit 4 comprises an evaporator 41 set in thermal communication with said cooling chamber 3 so as to subtract heat (directly or indirectly) from the cooling chamber 3.

A solute with a higher vapour pressure and a solvent with a lower vapour pressure circulate in the absorption refrigeration circuit 4. The solute typically consists of ammonia or lithium bromide. The solvent consists of water.

The absorption refrigeration circuit 4 comprises a vaporising zone 42 for vaporisation of the solute from a mixture comprising said solute and said solvent. In fact, following heating by the burner 211, the more volatile component (usually the ammonia) is released.

The refrigeration circuit 4 further comprises a condenser 43 in which the condensing of said solute coming from said vaporising zone 42 takes place.

As mentioned previously, the refrigeration circuit 4 comprises said evaporator 41 in which there is evaporation of said solute coming from the condenser 43. A lamination member 46 is conveniently present between the evaporator 41 and the condenser 43. The evaporator 41 is in thermal communication with said cooling chamber 3 (directly or by means of interposed working fluids). The evaporation of said solute is accompanied by the subtraction of heat from the cooling chamber 3.

The vaporising zone 42 comprises a channel 421 for passage of said solute-solvent mixture in said heat sink 212 and/or a tank 422 located downstream of said passage channel 421.

Suitably, the refrigeration circuit 4 comprises an absorber 45 in which the solute coming from the evaporator 41 is diluted in the solvent coming from the tank 422 (on its way from the tank 422, the solvent passes through a lamination member 44). The heat sink 212 is interposed between said absorber 45 and said tank 422, considering a direction of advancement of said mixture along the refrigeration circuit 4. In the preferred embodiment, a pump 47 is operatively interposed between said absorber 45 and said heat sink 212.

With reference to the embodiment illustrated in Figure 1, the operation of the refrigeration circuit mentioned previously is summarised herein below. The solute is separated from the solvent in the vaporising zone 42 by virtue of the heat supplied to the solute-solvent mixture by the heating means 21. The solute undergoes condensation in the condenser 43 and subsequently evaporates in the evaporator 41, subtracting heat from the cooling chamber 3. The solute coming from the evaporator 41 then flows into the absorber 45, in which it is mixed with the excess solvent coming from the vaporising zone 42. The solute-solvent mixture is then sent into the vaporising zone 42 by means of the pump 47 and the cycle is repeated.

The system 1 comprises means 5 for evacuating the water vapour generated by the combustion of oxyhydrogen in said burner 211. This water vapour, suitably re-condensed, could possibly be reutilised as a source liquid for the production of oxyhydrogen.

The system 1 comprises a sterilising tunnel 6. The tunnel 6 thus comprises:
- said sterilising chamber 2;
- said cooling chamber 3, which comprises a compartment 30 for refrigeration of the containers.

The cooling chamber 3 comprises means 31 for introducing a refrigerant fluid flow in said compartment 30. Suitably, this refrigerant fluid flow is cooled by the evaporator 41. The introducing means 31 for introducing the refrigerant fluid flow comprises a flow inlet for this purpose. This refrigerant fluid flow is suitably recirculated in said cooling chamber 3. In this regard, the recirculated refrigerant fluid flow is suitably filtered (usually by means of absolute filters).

The tunnel 6 advantageously also comprises said storage chamber 7. The chamber 7 is located upstream of said cooling chamber 3.

The system 1 further comprises means for taking said refrigerated fluid flow from the cooling chamber 3 and directing it into said storage chamber 7 (this solution is not illustrated). This brings about a pre-heating of said flow of sterile air.

The system 1 suitably comprises sterilising means by heating the cooling chamber 3. For example the sterilising means could comprise one or more electric heating elements, but it could also comprise means that draws heat from the oxyhydrogen burner 21, transferring the heat into said cooling chamber 3 (for example a conduit in which a working fluid circulates) or it could comprise an oxyhydrogen burner specifically dedicated to this. However, this sterilising means is not intended for the sterilisation or depyrogenation of the containers, but rather for the sterilisation of at least part of the cooling chamber 3.

The invention thus conceived enables many advantages to be achieved. First of all, it permits savings in terms of costs. In fact, the production of oxyhydrogen has lower production costs than those resulting from the consumption of electric energy through electric heating elements.

An important further advantage is the fact that high voltages are no longer needed to permit the heating elements to supply the heat required. Another important advantage is related to the fact that the combustion of oxyhydrogen only generates water vapour, which does not create problems relating to disposal of toxic products of combustion or problems concerning contamination of the phials or vials by such combustion products.

The invention thus conceived is susceptible to numerous modifications and variants, all of which falling within the scope of the inventive concept which characterises it. Moreover, all the details may be replaced with other technically equivalent elements. All materials used, as well as the dimensions and proportions, may in practice be of any type, according to requirements.

## Claims

1. A system for sterilising containers, comprising:
- a sterilising chamber (2) comprising a positioning zone (20) for the positioning of the containers;
- heating means (21) for heating a first fluid flow that envelops the containers placed in said positioning zone (20) so as to sterilise them; **characterised in that** it comprises supply means (210) for supplying oxyhydrogen; the heating means (21) comprising an oxyhydrogen burner (211) located downstream of the supply means (210).

2. The system according to claim 1, **characterised in that** the heating means (21) comprises a heat sink (212) heated by said burner (211) and in thermal communication with said first fluid flow.

3. The system according to claim 2, **characterised in that** said heat sink (212) is found in a first compartment (213) external to the positioning zone (20); said system comprising a filter that separates said first compartment (213) from said positioning zone (20) for the positioning of the containers, the first fluid flow traveling through said filter to pass from the first compartment (213) to the positioning zone (20).

4. The system according to any one of the preceding claims, **characterised in that** it comprises:
- a cooling chamber (3) for cooling the containers, said cooling chamber (3) being located downstream of the sterilising chamber (2) with respect to the direction of advancement of the containers;
- an absorption refrigeration circuit (4) comprising an evaporator (41) set in thermal communication with said cooling chamber (3) so as to subtract heat from the cooling chamber (3).

5. The system according to claim 4, **characterised in that** a solute with a higher vapour pressure and a solvent with a lower vapour pressure circulate in the absorption refrigeration circuit (4);
said absorption refrigeration circuit (4) comprising:
- a vaporising zone (42) for vaporisation of the solute from a mixture comprising said solute and said solvent;
- a condenser (43) wherein the condensing of said solute coming from said vaporising zone (42) takes place;
there being evaporation in the evaporator (41) of said solute coming from the condenser (43) and subtraction of heat from the cooling chamber (3).

6. The system according to claim 5, when directly or indirectly dependent on claim 2, **characterised in that** the vaporising zone (42) comprises a channel (421) for passage of said solute-solvent mixture in said heat sink (212) and/or a tank (422) located downstream of said passage channel (421) along a direction of advancement of said mixture.

7. The system according to claim 6, **characterised in that** the refrigeration circuit (4) comprises an absorber (45) wherein the solute coming from the evaporator (41) is diluted in the solvent coming from the tank (422); said mixture receiving heat from said heat sink (212), said heat sink (212) being interposed between said absorber (45) and said vaporising zone (42) with respect to a direction of advancement of said mixture along the refrigeration circuit (4).

8. The system according to claim 2 or 3, or according to claim 4 or 5 when directly or indirectly dependent on claim 2, **characterised in that** said heat sink (212) comprises at least one channel (421) for passage of said solute-solvent mixture, said passage channel (421) being integrated in the refrigeration circuit (4).

9. The system according to claim 2 or 3 or 6 or 7 or 8 or according to claim 4 or 5 when directly or indirectly dependent on claim 2, **characterised in that** said heat sink (212) is made of aluminium alloy.

## Patentansprüche

1. System zum Sterilisieren von Behältern, umfassend:
- eine Sterilisationskammer (2), umfassend eine Positionierungszone (20) zum Positionieren der Behälter;
- Heizmittel (21) zum Erhitzen eines ersten Fluidstroms, der die Behälter umhüllt, die in der Positionierungszone (20) positioniert sind, sodass diese sterilisiert, werden, **dadurch gekennzeichnet, dass** es Zuführmittel (210) zum Zuführen von Knallgas umfasst, wobei die Heizmittel (21) einen Wasserstoff-Sauerstoff-Brenner (211) umfassen, der nach den Zuführmitteln (210) angeordnet ist.

2. System nach Anspruch 1, **dadurch gekennzeichnet, dass** die Heizmittel (21) einen Wärmeableiter (212) umfassen, der vom Brenner (211) erhitzt wird und in thermischer Kommunikation mit dem ersten Fluidstrom steht.

3. System nach Anspruch 2, **dadurch gekennzeichnet, dass** der Wärmeableiter (212) in einer ersten Unterteilung (213) befindlich ist, die außerhalb der Positionierungszone (20) angeordnet ist, wobei das System einen Filter umfasst, der die erste Unterteilung (213) von der Positionierungszone (20) zum Positionieren der Behälter trennt, wobei der erste Fluidstrom von der ersten Unterteilung (213) durch den Filter zur Positionierungszone (20) strömt.

4. System nach einem der vorhergehenden Ansprüche, **dadurch gekennzeichnet, dass** es umfasst:
- eine Kühlkammer (3) zum Kühlen der Behälter, wobei die Kühlkammer (3) zur Vorschubrichtung der Behälter nach der Sterilisationskammer (2) angeordnet ist;
- einen Absorptionskühlkreislauf (4), umfassend einen Verdampfer (41), der in thermischer Kommunikation mit der Kühlkammer (3) steht, sodass Hitze aus der Kühlkammer (3) entzogen wird.

5. System nach Anspruch 4, **dadurch gekennzeichnet, dass** eine gelöste Substanz mit einem höheren Dampfdruck und ein Lösungsmittel mit einem niedrigeren Dampfdruck im Absorptionskühlkreislauf (4) zirkulieren; wobei der Absorptionskühlkreislauf (4) umfasst:
- eine Verdunstungszone (42) zum Verdunsten der gelösten Substanz aus einer Mischung, umfassend die gelöste Substanz und das Lösungsmittel;
- einen Verflüssiger (43), in dem das Verflüssigen der aus der Verdunstungszone (42) strömenden gelösten Substanz stattfindet;
wobei im Verdampfer (41) das Verdampfen der aus dem Verflüssiger (43) strömenden gelösten Substanz und das Entziehen von Hitze aus der Kühlkammer (3) stattfindet.

6. System nach Anspruch 5, wenn unmittelbar oder mittelbar abhängig von Anspruch 2, **dadurch gekennzeichnet, dass** die Verdunstungszone (42) einen Kanal (421) für das Durchströmen des Gemischs aus gelöster Substanz und Lösungsmittel im Wärmeableiter (212) und/oder einem Tank (422) umfasst, befindlich nach dem Durchströmungskanal (421) entlang einer Vorschubrichtung der Mischung.

7. System nach Anspruch 6, **dadurch gekennzeichnet, dass** der Kühlkreislauf (4) einen Absorber (45) umfasst, in dem die aus dem Verdampfer (41) strömende gelöste Substanz im aus dem Tank (422) strömenden Lösungsmittel verdünnt wird, wobei die Mischung Hitze vom Wärmeableiter (212) empfängt, wobei der Wärmeableiter (212) zwischen dem Absorber (45) und der Verdunstungszone (42) zur Vorschubrichtung der Mischung entlang des Kühlkreislaufs (4) angeordnet ist.

8. System nach Anspruch 2 oder 3 oder nach Anspruch 4 oder 5, wenn unmittelbar oder mittelbar von Anspruch 2 abhängig, **dadurch gekennzeichnet, dass** der Wärmeableiter (212) mindestens einen Kanal (421) für das Durchströmen der Mischung aus gelöster Substanz und Lösungsmittel umfasst, wobei der Durchströmungskanal (421) in den Kühlkreislauf (4) integriert ist.

9. System nach Anspruch 2 oder 3 oder 6 oder 7 oder 8 oder nach Anspruch 4 oder 5, wenn unmittelbar oder mittelbar von Anspruch 2 abhängig, **dadurch gekennzeichnet, dass** der Wärmeableiter (212) aus einer Aluminiumlegierung gefertigt ist.

## Revendications

1. Système de stérilisation de récipients, comprenant :
- une chambre de stérilisation (2) comprenant une zone de positionnement (20) pour le positionnement des récipients ;
- des moyens de chauffage (21) servant à chauffer un premier écoulement fluidique enveloppant les récipients placés dans ladite zone de positionnement (20) de manière à les stériliser ; **caractérisé en ce qu'**il comprend des moyens d'alimentation (210) pour alimenter en oxyhydrogène; les moyens de chauffage (21) comprenant un brûleur oxhydrique (211) situé en aval des moyens d'alimentation (210).

2. Système selon la revendication 1, **caractérisé en ce que** les moyens de chauffage (21) comprennent un dissipateur thermique (212) chauffé par ledit brûleur (211) et en communication thermique avec ledit premier écoulement fluidique.

3. Système selon la revendication 2, **caractérisé en ce que** ledit dissipateur thermique (212) se trouve dans un premier compartiment (213) extérieur à la zone de positionnement (20) ; ledit système comprenant un filtre qui sépare ledit premier compartiment (213) de ladite zone de positionnement (20) pour le positionnement des récipients, le premier écoulement fluidique passant à travers ledit filtre pour passer du premier compartiment (213) à la zone de positionnement (20).

4. Système selon l'une quelconque des revendications précédentes, **caractérisé en ce qu'**il comprend :
- une chambre de refroidissement (3) servant à refroidir les récipients, ladite chambre de refroidissement (3) étant située en aval de la chambre de stérilisation (2) par rapport à la direction de progression des récipients ;
- un circuit frigorifique à absorption (4) comprenant un évaporateur (41) mis en communication thermique avec ladite chambre de refroidissement (3) de manière à soustraire de la chaleur de la chambre de refroidissement (3).

5. Système selon la revendication 4, **caractérisé en ce qu'**un soluté ayant une pression de vapeur supérieure et un solvant ayant une pression de vapeur inférieure circulent dans le circuit frigorifique à absorption (4) ;
ledit circuit frigorifique à absorption (4) comprenant :
- une zone de vaporisation (42) du soluté à partir d'un mélange comprenant ledit soluté et ledit solvant ;
- un condensateur (43) dans lequel a lieu la condensation dudit soluté provenant de ladite zone de vaporisation (42) ;
de l'évaporation existant dans l'évaporateur (41) dudit soluté provenant du condensateur (43) et de la soustraction de chaleur de la chambre de refroidissement (3).

6. Système selon la revendication 5, lorsqu'elle dépend directement ou indirectement de la revendication 2, **caractérisé en ce que** la zone de vaporisation (42) comprend un canal (421) servant au passage dudit mélange soluté-solvant dans ledit dissipateur thermique (212) et/ou d'un réservoir (422) situé en aval dudit canal de passage (421) le long d'une direction de progression dudit mélange.

7. Système selon la revendication 6, **caractérisé en ce que** le circuit frigorifique (4) comprend un absorbeur (45) dans lequel le soluté provenant de l'évaporateur (41) est dilué dans le solvant provenant du réservoir (422) ; ledit mélange recevant de la chaleur dudit dissipateur thermique (212), ledit dissipateur thermique (212) étant interposé entre ledit absorbeur (45) et ladite zone de vaporisation (42) par rapport à une direction de progression dudit mélange le long du circuit frigorifique (4).

8. Système selon les revendications 2 ou 3, ou selon les revendications 4 ou 5 lorsqu'elles dépendent directement ou indirectement de la revendication 2, **caractérisé en ce que** ledit dissipateur thermique (212) comprend au moins un canal (421) pour le passage dudit mélange soluté-solvant, ledit canal de passage (421) étant intégré dans le circuit frigorifique (4).

9. Système selon les revendications 2 ou 3 ou 6 ou 7 ou 8 ou selon les revendications 4 ou 5 lorsqu'elles dépendent directement ou indirectement de la revendication 2, **caractérisé en ce que** ledit dissipateur thermique (212) est constitué d'un alliage d'aluminium.
